# EUROPÄISCHE PATENTSCHRIFT

(11) **EP 0 851 734 B1**
(45) Veröffentlichungstag und Bekanntmachung des Hinweises auf die Patenterteilung: **11.07.2001**
(21) Anmeldenummer: 95943220.4
(22) Anmeldetag: 27.12.1995
(51) Int. Cl.: A23F 3/14, A23F 3/06, A61K 35/78, A23F 3/36

(54) **KAPSELN, ENTHALTEND GEFRIERGETROCKNETE, GEPULVERTE GRÜNE TEEBLÄTTER**
CAPSULES CONTAINING FREEZE-DRIED POWDERED GREEN TEA LEAVES
CAPSULES RENFERMANT DES FEUILLES DE THE VERT EN POUDRE LYOPHILISE

(30) Priorität: 22.08.1995 DE 19530868
(43) Veröffentlichungstag der Anmeldung: 08.07.1998
(73) Patentinhaber: Freeze-Dry Foods GmbH, 48268 Greven (DE)
(72) Erfinder: ROHDEWALD, Peter, D-48341 Altenberge (DE)
(74) Vertreter: Huber, Bernhard, Dipl.-Chem.
(86) Internationale Anmeldenummer: EP9505149
(87) Internationale Veröffentlichungsnummer: WO9707685

(56) Entgegenhaltungen:
- DE-A- 3 414 767
- CHEMICAL ABSTRACTS, vol. 111, no. 3, 17.Juli 1989 Columbus, Ohio, US; abstract no. 17708, & JP,A,63 267 726 (TERUMO CO) 4.November 1988
- DATABASE WPI Week 8401 Derwent Publications Ltd., London, GB; AN 84-002818 & JP,A,58 198 246 (TAIYO KAGAKU KK)
- DATABASE WPI Week 9147 Derwent Publications Ltd., London, GB; AN 91-343136 & JP,A,03 228 646 (NIKEN FOOD KK) , 9.Oktober 1991
- DATABASE WPI Week 8932 Derwent Publications Ltd., London, GB; AN 89-230739 & JP,A,01 165 341 (ISSHIKI IKKO-DO KK) , 29.Juni 1989
- PATENT ABSTRACTS OF JAPAN vol. 12 no. 446 (C-546) ,24.November 1988 & JP,A,63 169933 (ATSUSHI SUGANUMA) 13.Juli 1988,
- DATABASE WPI Week 9532 Derwent Publications Ltd., London, GB; AN 95-241233 & CN,A,1 088 738 (ZHENG C) , 6.Juli 1994
- DATABASE WPI Week 7942 Derwent Publications Ltd., London, GB; AN 79-76387b & JP,A,54 116 394 (TOYO PULP KK)
- DATABASE WPI Week 9203 Derwent Publications Ltd., London, GB; AN 92-019297 & JP,A,03 266 954 (GOTO Y) , 27.November 1991

## Beschreibung

Die Erfindung betrifft ein Verfahren zur Herstellung eines Präparates, welches die Polyphenole des grünen Tees (Thea chinensis) in leicht verfügbarer, nicht oxidierter Form enthält, daß dadurch gekennzeichnet ist,daß man frische grüne Teeblätter abkühlt bis die Aktivität der enthaltenen Phenoloxidasen auf höchstens 1 % des Wertes bei Normaltemperatur gefallen ist und gleichzeitig oder unmittelbar danach das als Reaktionsmedium wirksame Wasser entfernt und nach diesem Verfahren hergestellte Produkte.

Es ist bekannt, daß unfermentierter (grüner) Tee neben der Wirkung als Genußmittel auch pharmakologische Eigenschaften aufweist, die geeignet sind, das Entstehen von Erkrankungen dadurch zu verhindern, daß einerseits die antioxidative Kapazität des menschlichen oder tierischen Organismus erhöht wird und weiterhin das vaskuläre System, insbesondere das Kapillarsystem geschützt wird.

Die antioxidativen Eigenschaften des unfermentierten Tees lassen sich zurückführen auf die Fähigkeit der in den Blättern von Thea chinensis enthaltenen Polyphenole, speziell der Gallocatechine, freie Radikale zu inaktivieren. Diesbezügliche Übersichten finden sich bei Lit. 1 des beigefügten Literaturverzeichnisses.

In engem Zusammenhang mit der Bindung freier Radikale steht die mehrfach nachgewiesene inaktivierende Wirkung des unfermentierten Tees gegenüber carcinogenen und procarcinogenen Substanzen (Lit. 2). Ein antibakterieller und bakterizider Effekt des unfermentierten Tees wurde ebenfalls nachgewiesen (Lit. 3). Schließlich zeigte sich in Tierversuchen auch eine Senkung des Plasmacholesterins (Lit. 4).

Zur Nutzung der Polyphenole der Teeblätter wurden bisher verschiedene Extraktionsverfahren angewandt, um die wasserlöslichen Bestandteile möglichst quantitativ zu extrahieren und einen wasserlöslichen Extrakt herzustellen, wobei als Ausgangsmaterial sowohl fermentierter als auch unfermentierter Tee Verwendung fand (Lit. 5).

Aus CA, Vol. 111, Nr. 3, Abstract Nr. 17708 ist ein Verfahren zur Extraktion von grünen Teeblättern mit organischen Lösungsmittel bekannt, bei dem die Teeblätter vor der Extraktion mit heißem Wasser gefriergetrocknet werden und der wässrige Extrakt mit organischen Lösungsmittel extrahiert wird. Die Oxidation der Polyphenole lässt sich auf diese Weise nicht verhindern. Weiter ist aus Database WPI, Week 8401, AN 84-002818 ein Verfahren bekannt, bei dem Teeblätter bei tiefer Temperatur pulverisiert, das Pulver mit einem wasserlöslichen Mittel granuliert und mit Heißluft getrocknet wird. Aus Database WPI, Week 9147, Derwent AN 91-343136 ist schließlich auch ein Verfahren zur Herstellung von löslichem Teepulver bekannt, bei dem spät gepflückte Teeblätter bei einer Temperatur unter 50 °C pulverisiert und mit gefriergetrocknetem Extrakt aus früh gepflückten Teeblättern gemischt wird. Auch für diese Verfahren gilt, dass eine Oxidation der Polyphenole so nicht vermieden werden kann.

Die Oxidation wertbestimmender Bestandteile der Teeblätter beginnt kurz nach der Ernte durch Phenoloxidasen. Auch nach Inaktivierung der Phenoloxidasen durch Erhitzen erfolgt weiterhin eine Oxidation der Polyphenole. Die Fermentierung des Tees hat schließlich zur Folge, daß die Oxidation gesteuert zur Bildung bestimmter Aromastoffe genutzt wird.

Der Erfindung liegt daher die Aufgabe zugrunde, die gesamte Palette der Inhaltsstoffe der frischen grünen Teeblätter möglichst weitgehend qualitativ wie quantitativ intakt zu erhalten, gleichzeitig aber für den Verbraucher leicht verfügbar zu machen. Insbesondere ist es eine Aufgabe der Erfindung die qualitativ und quantitativ intakt erhaltenen Inhaltsstoffe in eine Form zu bringen, die dem Verbraucher die Möglichkeit bietet, diese Inhaltsstoffe der Teeblätter entweder in Form eines Aufgusses zu sich zu nehmen oder aber die Teeblätter selbst als solche in trockener Form einzunehmen.

Gelöst wird diese Aufgabe erfindungsgemäß dadurch, daß man die Aktivität der Phenoloxidasen zunächst durch Kühlung der frischen grünen Teeblätter minimiert und gleichzeitig oder unmittelbar darauf die enzymatische Umsetzung der Polyphenole durch Phenoloxidasen durch Entzug des Reaktionsmediums Wasser verhindert. Hierzu werden insbesondere die frischen grünen Teeblätter abgekühlt bis die Aktivität der enthaltenen Phenoloxidasen auf höchstens 1 % des Wertes bei Normaltemperatur gefallen ist und gleichzeitig oder unmittelbar danach das als Reaktionsmedium wirksame Wasser entfernt.

Vorzugsweise werden Kühlung und Wasserentzug gleichzeitig durch Lyophilisierung (Gefriertrocknung) vorgenommen. Die gefriergetrockneten unfermentierten Teeblätter werden danach in nicht oxidierender Atmosphäre fein gepulvert und anschließend wiederum unter Sauerstoffausschluß verpackt. Als nicht oxidierende Atmosphäre wird vorzugsweise Stickstoff- oder/und Edelgasatmosphäre verwendet. Jedoch können auch andere Atmosphärenzusammensetzungen verwendet werden, vorausgesetzt, sie üben keine oxidierende Wirkung auf die Polyphenole aus.

Die erfindungsgemäß hergestellen gefriergetrockneten unfermentierten Teeblätter in feingepulverter Form können direkt abgepackt oder vorher noch einer Behandlung zur Entziehung des Koffeins unterworfen werden. Die Koffeinentfernung erfolgt zweckmäßig durch Extraktion mit überkritischem CO₂, also unter Bedingungen, die hinsichtlich Druck und Temperatur über der kritischen Grenze liegen. Verfahren zur Extraktion von pflanzlichem Material mit überkritischem CO₂ sind dem Fachmann bekannt und bedürfen daher hier keiner näheren Erläuterung. Wird eine solche Koffeinextraktion durchgeführt, so läßt man vor der Abfüllung etwaige Reste an Extraktionsmittel einfach an der Atmosphäre sich verflüchtigen und nimmt dann die Abfüllung in gleicher Weise wie bei dem nicht der CO₂-Extraktion unterworfenen Pulver vor.

Die Abfüllung erfolgt vorzugsweise in biokompatible, in heißem Wasser vollkommen lösliche Kapseln unter den vorstehend genannten Bedingungen. Als besonders geeignet erwiesen sich Steckkapseln aus Gelatine oder Weichgelatinekapseln. Prinzipiell ist jedoch für diese Ausführungsform der Erfindung jedes Kapselmaterial verwendbar, welches sich in Wasser von 100°C ohne Rückstände auflöst und pharmazeutisch verträglich ist.

Die erfindungsgemäß erhaltenen pulverisierten Teeblätterpräparate können jedoch auch in anderer Form verpackt werden, solang nicht oxidierende und wasserfreie Bedingungen aufrechterhalten werden können. So kommt beispielsweise die Verpackung in Liposomen unter Anwendung wasserfreier Methoden und Schutzgasatmosphäre in Betracht. Geeignete Herstellungsmethoden für derartige Liposomenpräparate sind dem Fachmann bekannt. Aufgrund der einfachen Herstellung und vielseitigen Anwendungsmöglichkeit werden jedoch die erfindungsgemäß erhältlichen Kapseln bevorzugt und sind daher für sich ein Gegenstand der Erfindung. Die erfindungsgemäßen Kapseln bewirken einerseits einen Schutz vor der Oxidation des Teepulvers, dienen aber andererseits auch dem Zweck, dem Verbraucher eine geschmacksneutrale oder geschmacklich nach Wunsch eingestellte Alternative zur Einnahme des erfindungsgemäß herstellten Teepulvers als Suspension in heißem Wasser zu bieten.

Den erfindungsgemäßen Kapseln können zusätzlich Antioxidantien, Vitamine und Spurenelemente zugesetzt werden. Ebenso können geschmackskorrigierende wasserfreie Aromen, insbesondere Pflanzenbestandteile zugesetzt werden. Dabei werden bevorzugt zur Teeherstellung bekannte getrocknete Pflanzen in Pulverform verwendet wie z.B. Pfefferminz, Malve, Kamille u.dgl.

Als Nahrungsergänzungsmittel werden erfindungsgemäße Kapseln mit einem Inhalt von 100 bis 400 mg der erfindungsgemäß erhaltenen feingepulverten unfermentierten grünen Teeblätter 1 bis 3 mal täglich entweder als wäßrige Suspension oder in Kapselform eingenommen. Soll die länger anhaltende und langsam einsetzende belebende Wirkung der Teeblätter im Vordergrund stehen, so werden Kapseln mit 400 bis 800 mg erfindungsgemäß erhaltenen Teeblättermaterial gefüllt, die nach Bedarf entweder als wäßrige Suspension oder als Kapsel eingenommen werden.

Die folgenden Beispiele erläutern die Erfindung.

### Beispiel 1

Frisch gepflückte Blätter des Teestrauchs werden im Kühlwagen zur Gefriertrocknung transportiert und am gleichen Tag tiefgefroren und gefriergetrocknet. Die gefriergetrockneten Blätter werden unter Stickstoffbegasung gepulvert. Dieses Pulver wird entweder sofort unter Stickstoffbegasung in dicht schließende Behälter abgefüllt oder sofort unter Stickstoffbegasung in Gelatinesteckkapseln abgefüllt. Anstelle der Gelatinesteckkapseln können auch Weichgelatinekapseln verwendet werden.

### Beispiel 2

Gemäß Beispiel 1 hergestellte pulverförmige gefriergetrocknete Teeblätter werden zur Koffeinextraktion mit überkritischem CO₂ bei einer Temperatur von etwa 40°C und einem Druck von etwa 200 bar extrahiert. Das CO₂ wird nach Abscheidung des extrahierten Koffeins durch Entspannung unter den kritischen Punkt recyclisiert und nach erneuter Einstellung der Extraktionsbedingungen wird die Arbeitsweise so lange wiederholt, bis der Restgehalt an Koffein unter 5 % des Ausgangswertes gesunken ist. Das extrahierte Teepulver wird mit Stickstoff begast, bis die Kohlendioxidentwicklung aus dem Pflanzenmaterial beendet ist und dann unter Fortsetzung der Stickstoffbegasung wie in Beispiel 1 beschrieben in Kapseln abgefüllt.

### Literaturverzeichnis

- Lit. 1: Scavenging Effect of Extracts of Green Tea and Natural Antioxidants on Active Oxygen Radicals. Baoulu Z., Xiyojie L., Rungen H., Shujun Ch. and Xin W. Cell Biophysics 1989, 14: 175-182
- Lit. 2: Anticarcinogenic activity of green tea polyphenols. Komori A. et al., Jpn. J. Clin. Oncol. 1993, June 23 (3): 186-90
- Lit. 3: A Pilot Study of Japanese Green Tea as a Medicament: Antibacterial and Bactericidal Effects. Naoki H., Yoshinori M., Masato I., Toru M. and Hiroshi N. J. Endodontics 1991, 17 (3): 122-24
- Lit. 4: Effect of Green Tea Catechins on Plasma Cholesterol Level in Cholesterol-Fed Rats. Muramatsu K., Fukuyo M. and Hara Y., J. Nutr. Sci. Vitaminol 1986, 32:613-622
- Lit. 5: DE 43 34 734 A1
GB 13 29 612
NL 68 09 368
Dalgleish J. McN: Freeze-Drying for the Food Industries, Elsevier applied Science, 1990 S. 68/69

## Patentansprüche

1. Verfahren zur Herstellung eines Präparates, welches die Polyphenole des grünen Tees (Thea chinensis) in leicht verfügbarer, nicht oxidierter Form enthält,
dadurch gekennzeichnet,
daß man frische grüne Teeblätter abkühlt bis die Aktivität der enthaltenen Phenoloxidasen auf höchstens 1 % des Wertes bei Normaltemperatur gefallen ist und gleichzeitig oder unmittelbar danach das als Reaktionsmedium wirksame Wasser entfernt, die gekühlten und getrockneten grünen Teeblätter pulverisiert und unter nicht oxidierenden und wasserfreien Bedingungen verpackt.

2. Verfahren nach Anspruch 1, dadurch gekennzeichnet, daß man die Aktivitätsverminderung der Phenoloxidasen und den Wasserentzug gleichzeitig durch Lyophilisierung vornimmt.

3. Verfahren nach Anspruch 1 oder 2, dadurch gekennzeichnet, daß man in nicht oxidierender Atmosphäre arbeitet.

4. Verfahren nach Anspruch 3, dadurch gekennzeichnet, daß man unter Stickstoff- oder/und Edelgasatmosphäre arbeitet.

5. Verfahren nach einem der vorhergehenden Ansprüche, dadurch gekennzeichnet, daß man das erhaltene Pulver in biokompatible, in heißem Wasser von 100°C vollkommen lösliche Kapseln unter Sauerstoffausschluß abfüllt.

6. Verfahren nach Anspruch 5, dadurch gekennzeichnet, daß man dem Pulver genießbare Antioxidanten, Vitamine oder/und Spurenelemente zusetzt.

7. Verfahren nach Anspruch 5 oder 6, dadurch gekennzeichnet, daß man dem Pulver geschmackskorrigierende wasserfreie Aromen, insbesondere Pflanzenbestandteile, zusetzt.

8. Verfahren nach Anspruch 7, dadurch gekennzeichnet, daß man zur Teeherstellung bekannte Pflanzenpulver zusetzt.

9. Verfahren einem einem der Ansprüche 1 bis 9, dadurch gekennzeichnet, daß man die pulverisierten Teeblätter zur Koffeinentfernung extrahiert.

10. Verfahren nach Anspruch 9, dadurch gekennzeichnet, daß man die Extraktion mit hinsichtlich Druck und Temperatur überkritischem CO₂ durchführt.

## Revendications

1. Procédé pour la production d'une préparation, laquelle contient les polyphénols du thé vert (Thea Chinensis) sous une forme aisément disponible et non oxydée,
caractérisé en ce que l'on fait refroidir des feuilles de thé vert fraîches jusqu'à ce que l'activité des oxydases de phénol contenues soit tombée jusqu'à 1 % au maximum de la valeur à température normale, et l'on supprime simultanément ou immédiatement ensuite l'eau active à titre de milieu de réaction, on pulvérise les feuilles de thé vert refroidies et séchées, et on les emballe sous des conditions non oxydantes et déshydratées.

2. Procédé selon la revendication 1, caractérisé en ce que l'on procède à la réduction de l'activité des oxydases de phénol et à la déshydratation simultanément par lyophilisation.

3. Procédé selon l'une ou l'autre des revendications 1 et 2, caractérisé en ce que l'on opère dans une atmosphère non oxydante.

4. Procédé selon la revendication 3, caractérisé en ce que l'on opère sous atmosphère d'azote et/ou de gaz rares.

5. Procédé selon l'une des revendications précédentes, caractérisé en ce que l'on remplit la poudre obtenue dans des capsules biocompatibles, entièrement solubles dans l'eau chaude à 100°C.

6. Procédé selon la revendication 5, caractérisé en ce que l'on ajoute à la poudre des agents antioxydants, des vitamines et/ou des oligo-éléments comestibles.

7. Procédé selon l'une ou l'autre des revendications 5 et 6, caractérisé en ce que l'on ajoute à la poudre des arômes déshydratés, en particulier des extraits de plantes, destinés à corriger le goût.

8. Procédé selon la revendication 7, caractérisé en ce que l'on ajoute des poudres de plantes connues pour la production de thé.

9. Procédé selon l'une des revendications 1 à 9, caractérisé en ce que l'on extrait les feuilles de thé pulvérisées pour l'élimination de la caféine.

10. Procédé selon la revendication 9, caractérisé en ce que l'on exécute l'extraction avec du CO₂ sur-critique en ce qui concerne la pression et la température.

## Claims

1. A process for producing a preparation which contains the polyphenols of green tea (Thea chinensis) in a readily available non-oxidised form, characterised in that fresh green tea leaves are cooled until the activity of phenol oxidases contained therein has decreased to no more than 1 % of the value at normal temperature and simultaneously or immediately thereafter the water acting as reaction medium is removed, the cooled and dried green tea leaves are pulverised and packed under non-oxidising and water-free conditions.

2. A process according to Claim 1, characterised in that the reduction in the activity of the phenol oxidases and the removal of water is carried out simultaneously by freeze-drying.

3. A process according to Claim 1 or 2, characterised in that the operation proceeds in a non-oxidising atmosphere.

4. A process according to Claim 3, characterised in that the operation proceeds in a nitrogen and/or noble gas atmosphere.

5. A process according to any one of the preceding Claims, characterised in that the powder obtained is packed into biocompatible capsules completely soluble in hot water of 100°C, with the exclusion of oxygen.

6. A process according to Claim 5, characterised in that edible anti-oxidants, vitamins and/or trace elements are added to the powder.

7. A process according to Claim 5 or 6, characterised in that flavour-correcting water-free aromas, especially plant components, are added to the powder.

8. A process according to Claim 7, characterised in that plant powders known for the production of tea are added.

9. A process according to any one of Claims 1 to 9, characterised in that the pulverised tea leaves are extracted for the removal of caffeine.

10. A process according to Claim 9, characterised in that the extraction is carried out with CO₂ which is supercritical with regard to pressure and temperature.
